# EUROPEAN PATENT SPECIFICATION

(11) **EP 3 456 363 B1**
(45) Date of publication and mention of the grant of the patent: **04.11.2020**
(21) Application number: 17382614.0
(22) Date of filing: 18.09.2017
(51) Int. Cl.: A61L 27/22, A61L 27/36, A61L 27/38, A61L 27/44, A61L 27/46, A61L 27/52, C12N 5/0775

(54) **COMPOSITION FOR REGENERATING BONY TISSUE, METHOD FOR PREPARATION AND USE THEREOF**
ZUSAMMENSETZUNG ZUR REGENERATION VON KNOCHENGEWEBE, VERFAHREN ZUR HERSTELLUNG UND VERWENDUNG DAVON
COMPOSITION POUR LA RÉGÉNÉRATION DE TISSUS OSSEUX, PROCÉDÉ POUR SA PRÉPARATION ET SON UTILISATION

(43) Date of publication of application: 20.03.2019
(73) Proprietor: Banc De Sang I Teixits, 08005 Barcelona (ES)
(72) Inventor: VIVES ARMENGOL, Joaquim, 08302 Mataró (ES); OLIVER VILA, Irene, 08018 Barcelona (ES); VIVAS PRADILLO, Daniel, 08304 Mataro (ES); GRAU VORSTER, Marta, 17487 Castello d'Empuries (ES); GARCIA LOPEZ, Juan, 08029 Barcelona (ES)
(74) Representative: Durán-Corretjer, S.L.P.

(56) References cited:
- EP-A1- 2 361 971
- WO-A1-2015/058318
- EUN-JU KANG ET AL: "In Vitro and In Vivo Osteogenesis of Porcine Skin-Derived Mesenchymal Stem Cell-like Cells with a Demineralized Bone and Fibrin Glue Scaffold", TISSUE ENGINEERING PART A, vol. 16, no. 3, 1 March 2010 (2010-03-01), pages 815-827, XP55460186, ISSN: 1937-3341, DOI: 10.1089/ten.tea.2009.0439
- LIU GUANGPENG ET AL: "In vitro and in vivo evaluation of osteogenesis of human umbilical cord blood-derived mesenchymal stem cells on partially demineralized bone matrix", TISSUE ENGINEERING. PART A MAR 2010 LNKD- PUBMED:19839720,, vol. 16, no. 3, 18 January 2010 (2010-01-18), pages 971-982, XP002637110,
- YOICHI YAMADA ET AL: "Bone regeneration following injection of mesenchymal stem cells and fibrin glue with a biodegradable scaffold", JOURNAL OF CRANIO-MAXILLO-FACIAL SURGERY : OFFICIAL PUBLICATION OF THE EUROPEAN ASSOCIATION FOR CRANIO-MAXILLO-FACIAL SURGERY FEB 2003 LNKD- PUBMED:12553923,, vol. 31, no. 1, 1 February 2003 (2003-02-01), pages 27-33, XP002637111, DOI: 10.1016/S1010-5182(02)00143-9 [retrieved on 2003-01-22]

## Description

The present invention relates to the tissue engineering field. In particular, it refers to a composition for regenerating bone tissue comprising mesenchymal cells with osteogenic potential derived from the Wharton's jelly of the umbilical cord, bony particles and a hydrogel of fibrinogen, to the method for preparation and use thereof.

The confluence of cell therapy and tissue engineering fields allows the development of new tools based on the combination of three key components: 1) cells; 2) scaffolds; and 3) signals (Daar AS and Greenwood HL. "A proposed definition of regenerative medicine". J Tissue Eng Regen Med, 2007;1(3):179-84). Tissue engineering offers feasible solutions to traumatologists and orthopaedic surgeons in those cases where a medicine with osteogenic potential is needed in a hydrogel format that can adapt its shape to the tridimensional architecture of the fracture site or bony defect to treat. Nowadays, autologous and heterologous transplant of bony tissue, as well as the use of implants based on diverse types of biomaterials, represent the most common approaches in the clinics. Among them, the bony autograft sourced from the iliac crest is the preferred treatment option for a wide range of orthopaedic conditions, including the management of complex fractures or in non-union defects.

The use of autologous tissue as a vector for bony regeneration fulfils three key requirements: 1) supplies of cells with osteogenic potential, 2) offers structural support, and 3) contributes with growth factors that promote vascularisation and osteoinduction. However, from the mechanistic point of view, the use of autografts presents important disadvantages in the clinical practice. In the first place, the complete substitution of the damaged bony tissue is rarely achieved through the use of autografts. This situation can lead to the failure of the autograft at long term (Wheeler DL and Enneking WF. "Allograft bone decreases in strength in vivo over time". Clin Orthop Relat Res, 2005;(435):36-42). On the other hand, the surgical approach employed for the collection of autologous bone is not exempt of risks to the patient and it is highly associated with morbidity (Chou LB et al. "Stress fracture as a complication of autogenous bone graft harvest from the distal tibia". Foot Ankle Int, 2007;28(2):199-201). In this sense, the use of human bone from tissue banks may solve the problem of the morbidity associated to the autografts but, on the contrary, it lacks the benefits associated to the regenerative activity displayed by the cellular component, which has been demonstrated to be a key factor after its successful use in animal models and clinical cases (Caminal M, et al. "A reproducible method for the isolation and expansion of ovine mesenchymal stromal cells from bone marrow for use in regenerative medicine preclinical studies". J Tissue Eng Regen Med, 2016). In fact, autografts are predominantly used for treating large bony defects even when bony tissue from tissue bank is available. Unfortunately, this option is not valid for all, specially recidivist, patients and, as it has been discussed previously, it is not exempt of risks due to the procedure of tissue extraction. Patent document EP 2361971 A1, discloses a procedure for preparing a tissue engineering product for the regeneration of bone tissues. The invention relates to a product which mainly comprises expanded mesenchymal cells of bone origin immobilized on bone supports and combined with fibrin gels.

In order to provide a solution to this issue, the present invention discloses a tissue engineering composition the use of which results in a new therapeutic tool for traumatologists and orthopaedic surgeons. The efficacy of the use of the composition obtained by the method disclosed in the present invention is based on the use of cells with osteogenic potential combined with bony particles embedded in a hydrogel that, altogether, induce the regeneration of damaged bony tissue adapting itself to the diverse architecture of the defects.

Both fibrin gels and bony particles sourced from tissue banks that were used in the present invention display key features as a support for the repair of the damaged bone tissue and also as a supply of a jelly texture that allows spacial localisation of the cellular component with osteogenic potential and its adaptation to the morphology of the fracture site. Besides of being biocompatible and resorbable, such non-cellular components produce a biological environment that ensures the supply of nutrients to the cells and assures their regenerative function. Moreover, bony particles hold the capacity of osteoinduction and osteoconduction.

Fibrin is a commercially available product (e.g. Tisseel/Tissucol from Baxter) that can also be manufactured *in situ* on demand (e.g. through the Cryoseal system from Thermogenesis) for its use as a biological glue, either as autologous or allogeneic. Typically, fibrin is presented in a formulation of two components: 1) concentrate or purified fibrinogen, that is the precursor glycoprotein of fibrin, and 2) Factor XIII, thrombin and calcium, which are used for triggering the polymerisation reaction (Buchta C, et al. "Fibrin sealant produced by the CryoSeal FS System: product chemistry, material properties and possible preparation in the autologous preoperative setting". Vox Sang, 2004;86(4):257-62). Fibrin glue is a widely known product in the surgery field since the 90's (Carless PA, et al. "Systematic review of the use of fibrin sealant to minimize perioperative allogeneic blood transfusion". Br J Surg, 2002;89(6):695-703) due to the advantages after its use for rapid hemostasis (Mankad PS and Codispoti M. "The role of fibrin sealants in hemostasis". Am J Surg, 2001 ;182:21S-8S), acceleration of wound healing (Amrani DL et al. "Wound healing. Role of commercial fibrin sealants". Ann N Y Acad Sci, 2001 ;936:566-79), reduction of blood loss (Carless PA, et al. "Systematic review of the use of fibrin sealant to minimize perioperative allogeneic blood transfusion". Br J Surg, 2002;89(6):695-703), protection against bacterial infections (Currie LJ, et al. "The use of fibrin glue in skin grafts and tissue-engineered skin replacements: a review". Plast Reconstr Surg, 2001;108(6):1713-26) and its texture that makes possible to shape to the architecture of the application zone (Caminal M, et al. "A reproducible method for the isolation and expansion of ovine mesenchymal stromal cells from bone marrow for use in regenerative medicine preclinical studies". J Tissue Eng Regen Med, 2016). Since human fibrin is highly biocompatible and biodegradable, fibrin can be used as a potential vehicle for bioactive ingredients in tissue engineering strategies. In the present invention, it has been proved that cells with osteogenic potential maintain their features when combined with fibrinogen and, thus, the compatibility of fibrin and cells with osteogenic potential facilitate their implantation into the bony defects for *in situ* generation of new tissue, either through direct differentiation of administered cells with osteogenic potential or as a result of the paracrine activity of the cells that exert a "calling" effect or signaling at local and systemic levels (Liu G, et al. "In vitro and in vivo evaluation of osteogenesis of human umbilical cord blood-derived mesenchymal stem cells on partially demineralized bone matrix". Tissue Eng Part A, 2010;16(3):971-82) or as a result of the combination of both mechanisms.

In the present invention, the terms "bony particles" and "bony matrix" mean the same and are referred to a composition that provides the specific features and properties of bone.

In the present invention, the term "injectable bone" is referred to a mixture of mesenchymal cells with osteogenic potential, bone particles and a fibrinogen hydrogel that, in combination with thrombin, coagulates adapting its shape to the architecture of the bony defect to treat.

In the present invention, the terms "hydrogel" and "gel" mean the same and are referred to a tridimensional net of flexible chains, comprised of connected elements in a particular manner and swelled by a liquid.

The present invention discloses a composition for regenerating bony tissue that comprises one or more mesenchymal cells of the umbilical cord with osteogenic potential, bony particles and a fibrinogen hydrogel. More specifically, the fibrinogen hydrogel is presented as an injectable hydrogel. The supply of cells with osteogenic potential is obtained after expansion and/or induction (or priming) to osteogenic differentiation through culture of mesenchymal cells with osteogenic potential derived from the Wharton's jelly of the umbilical cord in osteoinducive conditions.

Preferably, said hydrogel is a fibrinogen hydrogel or, after the contact of fibrinogen with thrombin, is fibrin.

Preferably, said bony particles are deantigenised bone or deantigenised and demineralised bone.

In a particular embodiment, the size of the injury to treat will determine the final volume of injectable bone and therefore the number of mesenchymal cells of the umbilical cord with osteogenic potential and bony particles required for a suitable bony regeneration. Preferably, the mesenchymal cells of the umbilical cord with osteogenic potential resuspended in saline solution and the hydrogel of fibrinogen are present in a proportion of 1:1 (v/v). On the other hand, preferably the bony particles are present within the composition of the present invention in a concentration of at least 3.5% (v/v).

In a preferred embodiment, the total number of mesenchymal cells of the umbilical cord per volume of composition is comprised between 1 and 1x10⁹ cells/mL. Preferably, the total number of mesenchymal cells of the umbilical cord per volume of composition is comprised within 1x10² and 1x10⁹ cells/mL.

On the other hand, the present invention discloses a method for the preparation of a composition for regenerating bony tissue as mentioned above, which comprises the steps of:
a) Obtaining cells with osteogenic potential from mesenchymal cells of the umbilical cord.
b) Mixing one or more cells collected in a) and bony particles within a fibrinogen hydrogel.

Preferably, said hydrogel is a fibrinogen hydrogel or, after the contact of fibrinogen with thrombin, is fibrin.

Preferably, the aforementioned bony particles are made of deantigenised bone or deantigenised and demineralised bone.

In said method, mesenchymal cells of the umbilical cord with osteogenic potential resuspended in saline solution and the fibrinogen hydrogel are preferably present in a proportion 1:1 (v/v). On the other hand, the bony particles are present preferably in the composition of the invention in a concentration of at least 3.5% (v/v).

In a preferred embodiment, the total number of mesenchymal cells of the umbilical cord per volume of composition is comprised within 1 and 1x10⁹ cells/mL. Preferably, the total number of mesenchymal cells of the umbilical cord per volume of composition is comprised within 1x10² and 1x10⁹ cells/mL.

In another embodiment, said osteogenic hydrogel that we call "injectable bone" can be used directly, without being mixed with thrombin, to regenerate bony defects due to its hydrogel nature, which will let it spread adapting its shape to the defect architecture and the coagulation will take place *in situ* because of the thrombin present due to bleeding at the injury site.

Optionally, diluted thrombin 1:100 (v/v) can be used or thrombin together with saline solution that contains calcium in physiological saline solution for activating the coagulation of the fibrinogen *ex vivo,* in a proportion of "injectable bone": thrombin of 10:3 (v/v).

On the other hand, the present invention discloses the use of said composition for the treatment of the regeneration of bony tissue induction. In that case, the mentioned composition has to be dispensed within 5-6 minutes to the fracture site, before the clot had been assembled totally and the combination of cells with osteogenic potential, bony particles and fibrin had lost its texture of fluid hydrogel and had become in a jelly clot.

Preferred embodiments that exemplify the present invention in detail in reference to the figures are presented next.
Figure 1 is a diagram of the method of the present invention.
Figure 2 is an illustration of the composition of the present invention after adapting to the shape of a syringe. The edge of said syringe is cut in order to facilitate the intake of the hydrogel of fibrinogen first, and the outflow of the cylindrical jellified construct at the end of the process.

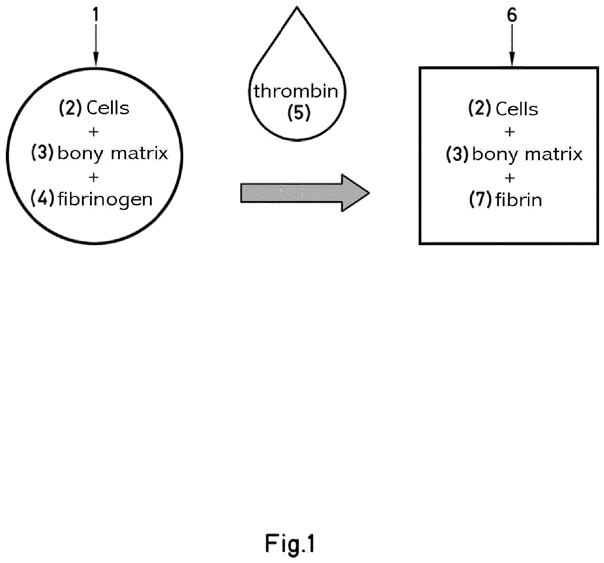

In a first embodiment, as it is shown in the figure 1, the method comprises an initial step of obtaining the osteogenic hydrogel -1-. Said osteogenic hydrogel -1-, called "injectable bone", contains cells -2-, bony matrix -3- and fibrinogen -4-. Afterwards, in a second step, said hydrogel is mixed with thrombin -5-creating a clot that adapts to the shape of the defect -6- that comprises said cells -2-, said bony matrix -3- and fibrin -7-.

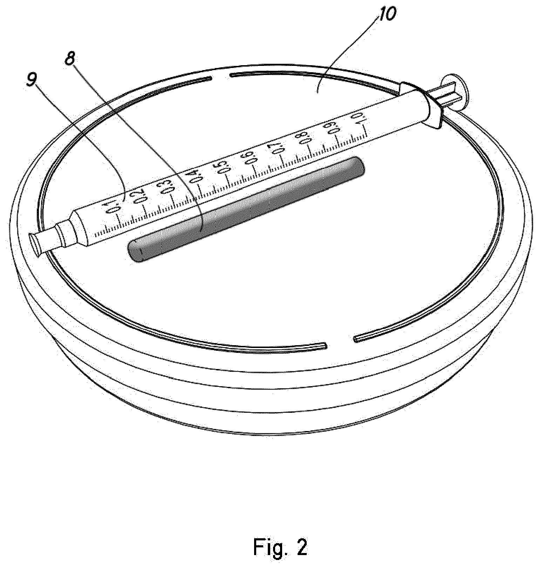

Figure 2 shows the cylindrical aspect of the injectable bone product adapted to the shape of a syringe of 1mL -8- after mixing mesenchymal cells expanded from the Wharton jelly of the umbilical cord, commercial fibrinogen, cadaveric, particulated, deantigenised and demineralised bone and commercial thrombin. Five minutes after performing the mixture, this was aspirated in a syringe -9- and was observed as the mixture clotted, adapting perfectly to the cylindrical architecture of said syringe -9-. Said syringe -9- and the product of injectable bone -8- were placed on a Petri dish -10-.

Hereinafter, the present invention is described with reference to examples, which however are not intended to limit the present invention.

### EXAMPLES

EXAMPLE 1: Method using mesenchymal cells derived from the Wharton's jelly of the umbilical cord.

In this case, mesenchymal cells expanded from the Wharton's jelly of the umbilical cord were produced following a Good Manufacturing Practices-validated process. The umbilical cord was disaggregated and cultured *in vitro,* allowing for the expansion of the MSC population. Those cells were kept in culture until reaching the needed number of cells in order to generate the constructs. In that particular case, cryoprecipitated fibrinogen obtained by the Cryoseal® FS System and commercial thrombin (Tissucol Duo, Baxter) were used. A cellular pellet was obtained and then resuspended in a volume of saline solution supplemented with 2% (w/v) of human albumin (Albutein®, Grifols). Then the same volume of cryoprecipitated fibrinogen through the Cryoseal® FS System was added. This mixture was combined with cadaveric, particulated, decellularised and deantigenised bone from tissue bank (diameter of particles comprised between 0.25-1mm) and commercial thrombin (Tissucol Duo, Baxter) diluted 1:100 in saline solution, as shown in figure 1. Within the first 5 minutes immediately after performing the mixture, this was aspirated into a 1mL syringe whose edge was previously cut, with the aim of simulating a cylindric bone defect.

On those conditions, the jelly mixture clotted just minutes after adapting perfectly its shape to the cyllindric architecture of the syringe, as shown in figure 2. The cellular dose applied in this case was 60x10⁶ MSC/cm³ (4.3x10⁶ MSC/mL) of bone.

Those cylinders were cut in a way that 0.2 cm³ constructs were obtained for the following *in vivo* verification of the osteogenic and angiogenic capacity of the constructs by means of the use of a model of athymic mouse that accepts the heterologous implantation of biological material. The constructs were implanted in a subcutaneous manner between the scapulas of the animals.

EXAMPLE 2: Method using mesenchymal cells derived from the Wharton's jelly of the umbilical cord for demonstrating the retention of the osteogenic potential and cellular viability (without the thrombin component).

In this example, the impact on cellular viability of both fibrin and the fibrinogen component alone as well as the retention of the osteogenic potential of the mesenchymal cells derived from the Wharton's jelly of the umbilical cord were tested *in vitro.* The fibrinogen component alone was evaluated, using both the commercial (Tissucol Duo, Baxter) as well as another one produced *in situ* using the Cryoseal® FS System (Thermogenesis). A strategy of coating the surface of the culture's vessels with fibrinogen was followed and then mesenchymal cells were seeded.

Surprisingly both fibrins maintained the cells alive and metabolically active and the fibrinogen component did not alter the osteogenic differentiation capacity of mesenchymal cells. The methodologies followed in this example included 1) determination of intracellular ATP as an indirect measure of the cellular metabolic state and 2) specific qualitative stainings of bony tissue markers, namely alkaline phosphatase enzyme and calcium depositions (stained with the Alizarin Red reactive) after a process of osteogenic differentiation induction of mesenchymal cells derived from the Wharton's jelly.

## Claims

1. Composition for regenerating bony tissue **characterized in that** it comprises one or more mesenchymal cells from the umbilical cord with osteogenic potential, bony particles and a fibrinogen hydrogel.

2. Composition according to claim 1, **characterized in that** said hydrogel consists of either fibrinogen or, after the addition of thrombin, fibrin.

3. Composition according to claim 1 or 2, **characterized in that** said bony particles are deantigenised or deantigenised/demineralised bone.

4. Composition according to any one of the preceding claims, **characterized in that** said mesenchymal cells from the umbilical cord with osteogenic potential resuspended in saline solution and said fibrinogen hydrogel are present in a proportion of 1:1 (v/v).

5. Composition according to any one of the preceding claims, **characterized in that** said bony particles are present in a concentration of at least 3.5% (v/v).

6. Composition according to any one of the preceding claims, **characterized in that** the total number of mesenchymal cells from the umbilical cord per volume of composition is ranged between 1x10² and 1x10⁹ cells/mL.

7. Method for the preparation of a composition to regenerate bony tissue, according to claims 1 to 6, comprising the steps of:
a) Collecting cells with osteogenic potential from mesenchymal cells from the umbilical cord.
b) Mixing one or more cells obtained in a) in fibrinogen and bony particles.

8. Method according to claim 7, **characterized in that** said mixture obtained in b) is also combined with thrombin, giving rise to fibrin.

9. Method according to claim 7, **characterized in that** further comprises the step of adding thrombin and saline solution containing calcium to the mixture obtained in b).

10. Method according to claims 7 to 9, **characterized in that** said hydrogel is either fibrinogen or, after mixing with thrombin, fibrin.

11. Method according to claims 7 to 10, **characterized in that** said bony particles are deantigenised or deantigenised/demineralised bone.

12. Method according to claims 7 to 11, **characterized in that** the total cell number per volume of composition is comprised between 1x10² and 1x10⁹ cells/mL.

13. Method according to claims 7 to 12, **characterized in that** said mesenchymal cells from the umbilical cord with osteogenic potential resuspended in saline solution and said fibrinogen hydrogel are present in a proportion of 1:1 (v/v).

14. Method according to claims 7 to 13, **characterized in that** said bony particles are present in the composition of the present invention in a concentration of at least 3.5% (v/v).

15. Composition, according to claims 1 to 6, for use in the induction of bony tissue regeneration.

## Patentansprüche

1. Zusammensetzung zum Regenerieren von Knochengewebe, **dadurch gekennzeichnet, dass** sie eine oder mehrere mesenchymale Zellen aus der Nabelschnur mit osteogenem Potential, Knochenpartikeln und ein Fibrinogenhydrogel umfasst.

2. Zusammensetzung nach Anspruch 1, **dadurch gekennzeichnet, dass** das Hydrogel aus entweder Fibrinogen oder, nach der Zugabe von Thrombin, aus Fibrin besteht.

3. Zusammensetzung nach Anspruch 1 oder 2, **dadurch gekennzeichnet, dass** die Knochenpartikel deantigenisierter oder deantigenisierter/demineralisierter Knochen sind.

4. Zusammensetzung nach einem der vorhergehenden Ansprüche, **dadurch gekennzeichnet, dass** die mesenchymalen Zellen aus der Nabelschnur mit osteogenem Potential, die in Kochsalzlösung resuspendiert sind, und das Fibrinogenhydrogel in einem Verhältnis von 1:1 (v/v) vorliegen.

5. Zusammensetzung nach einem der vorhergehenden Ansprüche, **dadurch gekennzeichnet, dass** die Knochenpartikel in einer Konzentration von mindestens 3,5 % (v/v) vorliegen.

6. Zusammensetzung nach einem der vorhergehenden Ansprüche, **dadurch gekennzeichnet, dass** die Gesamtzahl an mesenchymalen Zellen aus der Nabelschnur pro Volumen der Zusammensetzung in einem Bereich zwischen 1 x 10² und 1 x 10⁹ Zellen/ml liegt.

7. Verfahren zum Herstellen von einer Zusammensetzung, um Knochengewebe zu regenerieren, nach den Ansprüchen 1 bis 6, umfassend die folgenden Schritte:
a) Entnehmen von Zellen mit osteogenem Potential aus mesenchymalen Zellen aus der Nabelschnur.
b) Mischen von einer oder mehrerer Zellen, die in a) erhalten werden, in Fibrinogen und Knochenpartikel.

8. Verfahren nach Anspruch 7, **dadurch gekennzeichnet, dass** die Mischung, die in b) erhaltenen wird, auch mit Thrombin kombiniert wird, was zu Fibrin führt.

9. Verfahren nach Anspruch 7, **dadurch gekennzeichnet, dass** es ferner den Schritt des Zugebens von Thrombin und calciumhaltiger Kochsalzlösung zu der Mischung, die in b) erhalten wird, umfasst.

10. Verfahren nach den Ansprüchen 7 bis 9, **dadurch gekennzeichnet, dass** das Hydrogel entweder Fibrinogen oder, nach dem Mischen mit Thrombin, Fibrin ist.

11. Verfahren nach den Ansprüchen 7 bis 10, **dadurch gekennzeichnet, dass** die Knochenpartikel deantigenisierter oder deantigenisierter/demineralisierter Knochen sind.

12. Verfahren nach den Ansprüchen 7 bis 11, **dadurch gekennzeichnet, dass** die Gesamtzellenzahl pro Volumen der Zusammensetzung zwischen 1 x 10² und 1 x 10⁹ Zellen/ml liegt.

13. Verfahren nach den Ansprüchen 7 bis 12, **dadurch gekennzeichnet, dass** die mesenchymalen Zellen aus der Nabelschnur mit osteogenem Potential, die in Kochsalzlösung resuspendiert wurden, und das Fibrinogenhydrogel in einem Verhältnis von 1:1 (v/v) vorliegen.

14. Verfahren nach den Ansprüchen 7 bis 13, **dadurch gekennzeichnet, dass** die Knochenpartikel in der Zusammensetzung der vorliegenden Erfindung in einer Konzentration von mindestens 3,5 % (v/v) vorliegen.

15. Zusammensetzung nach den Ansprüchen 1 bis 6 zur Verwendung bei der Induktion einer Knochengeweberegeneration.

## Revendications

1. Composition pour la régénération de tissus osseux, **caractérisée en ce qu'**elle comprend une ou plusieurs cellules mésenchymateuses provenant du cordon ombilical ayant un potentiel osteogénique, des particules osseuses et un hydrogel de fibrinogène.

2. Composition selon la revendication 1, **caractérisée en ce que** ledit hydrogel consiste soit en fibrinogène, soit en fibrine après addition de thrombine.

3. Composition selon la revendication 1 ou la revendication 2, **caractérisée en ce que** lesdites particules osseuses sont en os déantigénisé ou en os déantigénisé / déminéralisé.

4. Composition selon l'une quelconque des revendications précédentes, **caractérisée en ce que** lesdites cellules mésenchymateuses provenant du cordon ombilical ayant un potentiel osteogénique et remises en suspension dans une solution saline, et ledit hydrogel de fibrinogène, sont présents selon une proportion de 1:1 (en volume).

5. Composition selon l'une quelconque des revendications précédentes, **caractérisée en ce que** lesdites particules osseuses sont présentes selon une concentration d'au moins 3.5 % (en volume).

6. Composition selon l'une quelconque des revendications précédentes, **caractérisée en ce que** le nombre total de cellules mésenchymateuses provenant du cordon ombilical en volume de composition est dans la plage comprise entre 1x10² et 1x10⁹ cellules par millilitre.

7. Procédé pour la préparation d'une composition pour régénérer les tissus osseux, selon les revendications 1 à 6, comprenant les étapes de :
a) collecter des cellules ayant un potentiel ostéogénique à partir de cellules mésenchymateuses provenant du cordon ombilical,
b) mélanger une ou plusieurs cellules obtenues en a) dans un fibrinogène et des particules osseuses.

8. Procédé selon la revendication 7, **caractérisé en ce que** ledit mélange obtenu en b) est également combiné avec de la thrombine, produisant l'apparition de fibrine.

9. Procédé selon la revendication 7, **caractérisé en ce qu'**il comprend en outre l'étape d'ajouter au mélange obtenu en b) de la thrombine et une solution saline contenant du calcium.

10. Procédé selon les revendications 7 à 9, **caractérisé en ce que** ledit hydrogel est soit du fibrinogène soit, après mélange avec de la thrombine, de la fibrine.

11. Procédé selon les revendications 7 à 10, **caractérisé en ce que** lesdites particules osseuses sont de l'os déantigénisé ou déantigénisé / déminéralisé.

12. Procédé selon les revendications 7 à 11, **caractérisé en ce que** le nombre total de cellules par volume de composition est compris entre 1x10² et 1x10⁹ cellules par millilitre.

13. Procédé selon les revendications 7 à 12, **caractérisé en ce que** lesdites cellules mésenchymateuses provenant du cordon ombilical avec un potentiel ostéogénique et remises en suspension dans une solution saline, et ledit hydrogel de fibrinogène, sont présents en une proportion de 1:1 (en volume).

14. Procédé selon les revendications 7 à 13, **caractérisé en ce que** lesdites particules osseuses sont présentes dans la composition de la présente invention selon une concentration d'au moins 3.5 % (en volume).

15. Composition, selon les revendications 1 à 6, pour utilisation dans l'induction de la régénération de tissus osseux.
